# EUROPEAN PATENT APPLICATION

(11) **EP 2 287 301 A2**
(43) Date of publication of application: **23.02.2011**
(21) Application number: 10171620.7
(22) Date of filing: 30.08.2005
(51) Int. Cl.: C12N 15/11, C07K 14/705, A61K 48/00, C12Q 1/68

(54) **Methods and compositions to inhibit P2X7 receptor expression**

(30) Priority: 31.08.2004 GB 0419295; 28.02.2005 GB 0504057
(62) Divisional of application: 05781262.0
(71) Applicant: Sylentis S.A.U., 28003 Madrid (ES)
(72) Inventor: Sesto, Angela, 28760 Madrid (ES); Roman, Jose P., 01009 Vitoria (ES); Jiminez, Ana I., 28922 Madrid (ES); Gascon, Irene, 28016 Madrid (ES); de Buitrago Gonzalo, Gonzalez, 28007 Madrid (ES); Jiminez, Maria Concepcion, 28903 Madrid (ES)
(74) Representative: Williams, Gareth Owen

(57) **Abstract**

Methods and compositions for the downregulation of P2X7 receptor expression or activity are disclosed. Preferred compositions comprise siNA. The methods and compositions are useful in the treatment of diseases characterised by increased P2X7 receptor activity, such as neuronal degeneration, Alzheimer's disease, inflammatory diseases, and some cancers.

## Description

### FIELD OF THE INVENTION

The present invention relates to methods and compositions for the treatment and/or the prevention of neuronal degeneration or other diseases related to high levels of expression or activity of P2X7 receptors (P2RX7). In preferred embodiments, the invention relates to the use of RNAi technology to downregulate the expression of P2RX7.

Methods and compositions are provided for the treatment of diseases related to high levels of P2RX7, which include, but are not limited to, neuronal degeneration, reperfusion or ischemia in stroke or heart attack, Alzheimer's disease, inflammatory diseases (such as rheumatoid arthritis, osteoarthritis, asthma, rhinitis, chronic obstructive pulmonary disease (COPD), inflammatory bowel disease (IBD) such as Crohn's disease), allergies, autoimmune diseases, cancer (such as leukaemia or non-melanoma skin cancer), skin-related conditions (such as psoriasis, eczema, alopecia), retinal diseases and treatment of pain of neuropathic and inflammatory origin.

### BACKGROUND OF THE INVENTION

### RNAi as a tool to downregulate gene expression

Gene targeting by homologous recombination is commonly used to determine gene function in mammals, but this is a costly and time-consuming process. Alternatively, the functions of many genes can be determined after mRNA inhibition with ribozyme or antisense technologies. Although successful in some situations these technologies have been difficult to apply universally. The advent of siRNA-directed "knockdown" has sparked a revolution in somatic cell genetics, allowing the inexpensive and rapid analysis of gene function in mammals.

Establishing a convenient and reliable method to knock-out gene expression at the mRNA level has been a recurrent theme in molecular biology over the last 15 years. In efforts to generate loss-of function cells or organisms, various molecules that included, for example, antisense sequences, ribozymes, and chimeric oligonucleotides have been tested, but the design of such molecules was based on trial and error, depending on the properties of the target gene. Moreover, the desired effects were difficult to predict, and often only weak suppression achieved (Braasch & Corey, 2002).

After the discovery of the phenomenon in plants in the early 1990s, in 1998 Andy Fire and Craig Mello for the first time demonstrated with the worm *Caenorhabditis elegans* that dsRNA (double-stranded RNA) may specifically and selectively inhibit gene expression in an extremely efficient manner (Fire *et al.,* 1998). In their experiment, the sequence of the first strand (the so-called *sense* RNA) coincides with that of the corresponding region of the target messenger RNA (mRNA). The second strand (*antisense* RNA) is complementary to this mRNA. The resulting dsRNA turned out to be far more (several orders of magnitude) efficient than the corresponding single-stranded RNA molecules (in particular, *antisense* RNA). Fire *et al.,* 1998 named the phenomenon RNAi for RNA *interference.* This powerful gene silencing mechanism has been shown to operate in several species among most phylogenetic phyla.

RNAi begins when an enzyme named DICER encounters dsRNA and chops it into pieces called small-interfering RNAs or siRNAs. This protein belongs to the RNase III nuclease family. A complex of proteins gathers up these RNA remains and uses their code as a guide to search out and destroy any RNAs in the cell with a matching sequence, such as target mRNA (for review see Bosher & Labouesse, 2000).

The RNAi phenomenon (Akashi *et al.,* 2001) might be summarized as follows:
- Step 1: dsRNA recognition and scanning process.
- Step 2: dsRNA cleavage through RNase III activity and production of siRNAs.
- Step 3: association of the siRNAs and associated factors in RISC complexes.
- Step 4: recognition of the complementary target mRNA.
- Step 5: cleavage of the target mRNA in the centre of the region complementary to the siRNA.
- Step 6: degradation of the target mRNA and recycling of the RISC complex.

In trying to apply the RNAi phenomenon as a technology for gene knockdown, it was soon realized that mammalian cells have developed various protective phenomena against viral infections that could impede the use of this approach. Indeed, the presence of extremely low levels of viral dsRNA triggers an interferon response, resulting in a global non-specific suppression of translation, which in turn triggers apoptosis (Williams, 1997, Gil & Esteban, 2000).

In 2000, a first attempt with dsRNA resulted in the specific inhibition of 3 genes (MmGFP under the control of the Elongation Factor 1a, E-cadherin, and c-mos) in the mouse oocyte and early embryo. Translational arrest, and thus a PKR response, was not observed as the embryos continued to develop (Wianny & Zernicka-Goetz, 2000). One year later, research at *Ribopharma AG* (Kulmbach, Germany) first demonstrated the functionality of RNAi in mammalian cells. Using short (20-24 base pairs) dsRNAs - which are called *SIRPLEX^{™}-* they specifically switched off genes even in human cells without initiating the acute-phase response. Similar experiments carried out later by other research groups (Elbashir *et al.,* 2001; Caplen *et al.,* 2001) further confirmed these results.

A year later, Paddison *et al.* (Paddison *et al,* 2002) tried to use small RNAs folded in hairpin structures to inhibit the function of specific genes. This work was inspired by previous studies showing that some genes in *Caenorhabditis elegans* naturally regulate other genes through RNAi by coding for hairpin-structured RNAs. Tested in a variety of normal and cancer human and mouse cell lines, short hairpin RNAs (shRNAs) are able to silence genes as efficiently as their siRNA counterparts. Moreover, shRNAs exhibit better reassociation kinetics *in vivo* than equivalent duplexes. Even more important, these authors generated transgenic cell lines engineered to synthesize shRNAs that exhibit a long-lasting suppressing effect throughout cell divisions (Eurogentec). Recently, another group of small RNAs (also comprised in the range of 21-25 nt) was shown to mediate downregulation of gene expression. These RNAs, known as small temporally regulated RNAs (stRNAs), have been described in *Caenorhabditis elegans* were they regulate timing of gene expression during development. It should be noted that stRNAs and siRNAs, despite obvious similarities, proceed through different modes of action (for review see Banerjee & Slack, 2002. In contrast with siRNAs, 22 nt long stRNAs downregulate expression of target mRNA after translational initiation without affecting mRNA integrity. Recent studies indicate that the two stRNAs first described in nematodes are the members of a huge family with hundreds of additional micro-RNAs (miRNAs) existing in metazoans (Grosshans & Slack, 2002).

Scientists have initially used RNAi in several systems, including *Caenorhabditis elegans,* Drosophila, trypanosomes, and various other invertebrates. Moreover, using this approach, several groups have recently presented the specific suppression of protein biosynthesis in different mammalian cell lines - specifically in HeLa cells - showing that RNAi is a broadly applicable method for gene silencing in vitro. Based on these results, RNAi has rapidly become a well recognized tool for validating (identifying and assigning) gene functions. RNA interference employing short dsRNA oligonucleotides will, moreover, permit to decipher the function of genes being only partially sequenced. RNAi will therefore become inevitable in studies such as:
- Inhibition of gene expression at the post-transcriptional level in eukaryotic cells. In this context, RNAi is a straight-forward tool to rapidly assess gene function and reveal null phenotypes.
- Development of the RNAi technology for use in post-implantation embryos.
- The predominant economic significance of RNA interference is established by its application as a therapeutic principle. As so, RNAi may yield RNA-based drugs to treat human diseases.

### Inhibition of high levels of P2RX7 to prevent disease.

P2X receptors are membrane ion channels that open in response to the binding of extracellular ATP (North, 2002). They are abundantly distributed, and functional responses are seen in neurons, glia, epithelia, endothelia, bone, muscle, and haematopoietic tissues.

The purinergic P2X7 receptors (P2RX7) are ligand-gated cation channels with a wide distribution that includes cells of the immune and haematopoietic system (Di Virgilio et al., 2001; North 2002). Two splice forms of P2RX7 corresponding to GenBank Accession Numbers NM_002562 and NM_177427 were initially identified. However, identification of seven variants of human P2RX7 which result from alternative splicing has recently been reported (Cheewatrakoolpong et al., 2005).

Activation of P2RX7 by brief exposure to extracellular ATP opens a channel that allows Ca²⁺ and Na⁺ influx and K⁺ efflux and that initiates a cascade of intracellular downstream events. These include the stimulation of phospholipase D (El-Moatassim & Dubyak, 1993; Gargett et al, 1996), the activation of membrane metalloproteases (Jamieson, et al., 1996; Gu et al, 1998; Sluyter & Wiley, 2002), and the stimulation of intracellular caspases, which eventually lead to the apoptotic death of the target cell (Ferrari et al, 1999; Humphreys et al, 2000). P2RX7 activation also leads to extensive membrane blebbing (Virginio et al., 1999), which is a typical morphological feature of the apoptotic process.

P2RX7 mediates fast excitatory transmission in diverse regions of the brain and spinal cord (North, 2002). ATP has recently been identified as a potent transmitter of astrocytic calcium signalling (Cotrina et al., 1998; Guthrie et al, 1999). Astrocytic calcium signalling seems to be a general mechanism by which astrocytes respond to a variety of stimuli including synaptic activity, transmitter exposure and traumatic injury (Fields & Stevens-Graham, 2002). By this means, local astrocytes transmit calcium signals to neurons within their own geographical microdomain. This ATP-dependent process of calcium wave propagation occurs in the brain as well as in the parenchyma of the spinal cord (Scemes et al., 2000, Fam et al., 2000), where it may have a role in extending local injury.

Preliminary observations indicate that traumatic injury triggers both ATP release and calcium signalling (Cook & McCleskey, 2002; Neary et al., 2003, Du *et al.,* 1999). The fact that P2RX7 is expressed in spinal cord neurons, including motor neurons (Deuchars *et al.,* 2001), and that P2RX7 is an ATP-gated cation channel whose activation directly mediates cell death (Di Virgilio *et al.,* 1998), target P2RX7 as good candidates to be inhibited for the prevention of traumatic injury consequences as well as of chronic trauma. Delivery of P2RX7 antagonists OxATP or PPADS to rats after acute impact injury significantly improved functional recovery and diminished cell death in the peritraumatic zone, reducing both the histological extent and functional sequelae of acute spinal cord injury (Wang et al., 2004).

A postischemic, time-dependent upregulation of the P2X7 receptor-subtype on neurons and glial cells has also been demonstrated, and suggests a role for this receptor in the pathophysiology of cerebral ischemia in vivo (Franke et al., 2004).

Parvathenani et al have shown a remarkable difference in the staining pattern for P2RX7 in brain slices of a transgenic mice model of Alzheimer's disease (AD) (Parvathenani et al, 2003). The intense staining for P2RX7 around plaques can be the result of up-regulation of the P2X7 receptor and/or aggregation of glia around plaques. The striking association in vivo between P2X7 receptor-positive cells and plaques in a transgenic mouse model of AD suggests that antagonists of P2RX7 could have therapeutic utility in treatment of AD by regulating pathologically activated microglia.

Extracellular ATP has proven to activate multiple downstream signalling events in a human T-lymphoblastoid cell line (Budagian et al., 2003). Both P2RX7 mRNA and protein have been detected in eight of eleven human haematopoietic cell lines in a non-lineage-specific manner (Zhang et al., 2004). Further, bone marrow mononuclear cell samples from 69 leukaemia and 9 myelodysplastic syndrome (MDS) patients (out of 87 and 10 patients, respectively) were P2RX7 positive at mRNA level. Moreover, both positive rates and relative expression levels were significantly higher in acute myelogenous leukaemia (AML), acute lymphoblastic leukaemia (ALL), chronic myelogenous leukaemia (CML), and MDS groups than in the normal donor group. After one course of standard induction therapies, the remission rate in high P2RX7 expression group was lower than that in either the P2RX7 negative group or the low P2RX7 expression group (Zhang et al., 2004). Expression and function of P2RX7 have also been associated with the clinical course of patients affected by chronic lymphocytic leukaemia (CLL) (Cabrini et al., 2005).

Dendritic cells (DC), which are central in the initiation of adaptive immune responses (Hart, 1997; Stockwin et al., 2000) express P2RX7 (Mutini et al., 1999; Berchtold et al., 1999; Ferrari et al., 2000). Further, it has been demonstrated that activation of P2RX7 in DC opens a cation-selective channel and leads to rapid and near complete shedding of CD23, the low affinity receptor for IgE (Sluyter & Wiley, 2002), which has an emerging role in chronic inflammatory diseases including rheumatoid arthritis (Bonnefy, 1996).

Electrophysiological data and mRNA analysis of human and mouse pulmonary epithelia and other epithelial cells indicate that multiple P2XRs are broadly expressed in these tissues and that they are active on both apical and basolateral surfaces (Taylor et al., 1999).

P2RX7 is also expressed on human cutaneous keratinocytes where it has a role in the signalling system for regulation of proliferation, differentiation and apoptosis of epidermis (Greig et al., 2003a; Greig et al., 2003b). Further to the above-mentioned effects, in response to ATP-binding P2RX7 contributes to the release of the biologically active inflammatory cytokine interleukin IL-1 beta, following activation of the cells of immune origin in which it is expressed such as LPS-primed macrophages (Verhoef et al., 2003). Involvement of P2RX7 in the production of the inflammatory response of monocytes/macrophages makes it a good target against cell-mediated autoimmune disorders such as psoriasis.

Expression of P2RX7 has also been detected on Muller glial cells from the human retina (Pannicke et al., 2000) as well as on pericytes of microvessels isolated from the rat retina, where they regulate the multicellular functional organization of the microvascular network (Kawamura et al., 2003). It has recently been demonstrated that stimulation of P2RX7 by means of agonists such as benzoylbenzoyl adenosine triphosphate (BzATP) elevates Ca2+ and kills retinal ganglion cells (Zhang et al., 2005).

Enhanced P2RX7 activity has been detected in human fibroblasts from diabetic patients, suggesting a possible pathogenetic mechanism for vascular damage in diabetes (Solini et al., 2004).

Experiments carried out with mice lacking P2RX7 demonstrate that inflammatory and neuropathic hypersensitivity is completely absent to both mechanical and thermal stimuli in mutant mice, whilst normal nociceptive processing is preserved (Chessell et al., 2005). The knockout animals were unimpaired in their ability to produce mRNA for pro-IL-1 beta, and cytometric analysis of paw and systemic cytokines from knockout and wild-type animals following adjuvant insult suggested a selective effect of the gene deletion on release of IL-1beta and IL-10. This piece of evidence, together with the fact that P2RX7 was upregulated in human dorsal root ganglia and injured nerves obtained from chronic neuropathic pain patients, served to hypothesise that P2RX7, via regulation of mature IL-1 beta production, plays a role in the development of pain of neuropathic and inflammatory origin (Chessell et al., 2005). Drugs which block this target may have the potential to deliver broad-spectrum analgesia.

The above-mentioned experimental evidence, therefore, points to inhibition of P2RX7 as an efficient treatment for diseases such as neuronal degeneration, reperfusion or ischemia in stroke or heart attack, Alzheimer's disease, inflammatory diseases (such as rheumatoid arthritis, osteoarthritis, asthma, rhinitis, chronic obstructive pulmonary disease (COPD), inflammatory bowel disease (IBD) such as Crohn's disease), allergies, autoimmune diseases, cancer (such as leukaemia, non-melanoma skin cancer), skin-related conditions (such as psoriasis, eczema, alopecia), retinal diseases and treatment of pain of neuropathic and inflammatory origin.

A novel approach to exert this inhibition is the down regulation of P2RX7 gene expression mediated by RNA interference (RNAi).

### BRIEF SUMMARY OF THE INVENTION

In the present invention we describe a method for the treatment and/or prevention of neuronal degeneration or other diseases related to high levels of P2RX7. The method is based on the downregulation of expression of one or more splice forms of the P2RX7 gene. Downregulation may be effected by the use of double stranded nucleic acid moieties, named siNA or small interfering NA that are directed at interfering with the mRNA expression of either one or more splicing forms of the P2RX7 gene. The siNA are preferably siRNA, although modified nucleic acids or similar chemically synthesised entities are also included within the scope of the invention.

Embodiments of the invention provide pharmaceutical compositions for use in the treatment of neuronal degeneration conditions and of other animal (including human) diseases related to high levels of P2RX7.

### DETAILED DESCRIPTION OF THE INVENTION

### Design of siNA

Although the mechanisms for RNAi remain unknown, the steps required to generate the specific dsRNA oligonucleotides are clear. It has been shown that dsRNA duplex strands that are 21-26 nucleotides in length work most effectively in producing RNA interference. Selecting the right homologous region within the gene is also important. Factors such as the distance from start codon, the G/C content and the location of adenosine dimers are important when considering the generation of dsRNA for RNAi. One consequence of this, however, is that one may need to test several different sequences for the most efficient RNAi and this may become costly.

In 1999, Tuschl *ef al.* deciphered the silencing effect of siRNAs showing that their efficiency is a function of the length of the duplex, the length of the 3'-end overhangs, and the sequence in these overhangs. Based on this founder work, Eurogentec recommends that the target mRNA region, and hence the sequence of the siRNA duplex, should be chosen using the following guidelines:
Since RNAi relies on the establishment of complex protein interactions, it is obvious that the mRNA target should be devoided of unrelated bound factors. In this context, both the 5' and 3' untranslated regions (UTRs) and regions close to the start codon should be avoided as they may be richer in regulatory protein binding sites. The sequence of the siRNA is therefore selected as follows:
   - In the mRNA sequence, a region located 50 to 100 nt downstream of the AUG start codon or upstream of stop codon is selected.
   - In this region, the following sequences are searched for: AA(N19), CA(N19).
   - The G/C percentage for each identified sequence is calculated. Ideally, the G/C content is 50 % but it must less than 70 % and greater than 30 %.
   - Preferably, sequences containing following repetitions are avoided: AAA, CCC, GGG, TTT, AAAA, CCCC, GGGG, TTTT.
   - An accessibility prediction according to the secondary structure of the mRNA is carried out as well.
   - A BLAST is also performed (i.e. NCBI ESTs database) with the nucleotide sequence fitting best the previous criteria to ensure that only one gene will be inactivated.

In order to maximize the result's interpretation, the following precautions should be taken when using siRNAs:
- Always test the sense and antisense single strands in separate experiments.
- Try a scramble siRNA duplex. This should have the same nucleotide composition as your siRNA but lack significant sequence homology to any other gene (including yours).
- If possible, knock-down the same gene with two independent siRNA duplexes to control the specificity of the silencing process.

Practically, each of the selected genes is introduced as a nucleotide sequence in a prediction program that takes into account all the variables described above for the design of optimal oligonucleotides. This program scans any mRNA nucleotide sequence for regions susceptible to be targeted by siRNAs. The output of this analysis is a score of possible siRNA oligonucleotides. The highest scores are used to design double stranded RNA oligonucleotides (typically 21 bp long, although other lengths are also possible) that are typically made by chemical synthesis.

In addition to siRNA, modified nucleotides may also be used. We plan to test several chemical modifications that are well known in the art. These modifications are aimed at increasing stability or availability of the siNA. Examples of suitable modifications are described in the publications referenced below, each of which is incorporated herein by reference.

Studies show that replacing the 3'-terminal nucleotide overhanging segments of a 21-mer siRNA duplex having two -nucleotide 3'-overhangs with deoxyribonucleotides does not have an adverse effect on RNAi activity. Replacing up to four nucleotides on each end of the siRNA with deoxyribonucleotides has been reported to be well tolerated, whereas complete substitution with deoxyribonucleotides results in no RNAi activity (Elbashir 2001). In addition, Elbashir et al. also report that substitution of siRNA with 2'-O- methyl nucleotides completely abolishes RNAi activity.

Affinity modified nucleosides as described in WO2005/044976 may be used. This publication describes oligonucleotides comprising nucleosides modified so as to have increased or decreased affinity for their complementary nucleotide in the target mRNA and/or in the complementary siNA strand.

GB2406568 describes alternative modified oligonucleotides chemically modified to provide improved resistance to degradation or improved uptake. Examples of such modifications include phosphorothioate internucleotide linkages, 2'-O-methyl ribonucleotides, 2'-deoxy-fluoro ribonucleotides, 2'-deoxy ribonucleotides, "universal base" nucleotides, 5-C-methyl nucleotides, and inverted deoxyabasic residue incorporation.

WO2004/029212 describes oligonucleotides modified to enhance the stability of the siRNA or to increase targeting efficiency. Modifications include chemical cross linking between the two complementary strands of an siRNA and chemical modification of a 3' terminus of a strand of an siRNA. Preferred modifications are internal modifications, for example, sugar modifications, nucleobase modifications and/or backbone modifications. 2'-fluoro modified ribonucleotides and 2'-deoxy ribonucleotides are described.

WO2005/040537 further recites modified oligonucleotides which may be used in the invention.

As well as making use of dsNA and modified dsNA, the present invention may use short hairpin NA (shNA); the two strands of the siNA molecule may be connected by a linker region, which may be a nucleotide linker or a non-nucleotide linker.

In addition to siNA which is perfectly complementary to the target region, degenerate siNA sequences may be used to target homologous regions. W02005/045037 describes the design of siNA molecules to target such homologous sequences, for example by incorporating non-canonical base pairs, for example mismatches and/or wobble base pairs, that can provide additional target sequences. In instances where mismatches are identified, non-canonical base pairs (for example, mismatches and/or wobble bases) can be used to generate siNA molecules that target more than one gene sequence. In a nonlimiting example, non-canonical base pairs such as UU and CC base pairs are used to generate siNA molecules that are capable of targeting sequences for differing targets that share sequence homology. As such, one advantage of using siNAs of the invention is that a single siNA can be designed to include nucleic acid sequence that is complementary to the nucleotide sequence that is conserved between homologous genes. In this approach, a single siNA can be used to inhibit expression of more than one gene instead of using more than one siNA molecule to target different genes.

Preferred siNA molecules of the invention are double stranded. A siNA molecule of the invention may comprise blunt ends, that is, ends that do not include any overhanging nucleotides. In one embodiment, an siNA molecule of the invention can comprise one or more blunt ends. In preferred embodiments, the siNA molecules have 3' overhangs. siNA molecules of the invention may comprise duplex nucleic acid molecules with 3' overhangs of n nucleotides (5≥*n*≥1). Elbashir (2001) shows that 21-nucleotide siRNA duplexes are most active when containing 3'-terminal dinucleotide overhangs.

Candidate oligonucleotides are further filtered for interspecies sequence conservation in order to facilitate the transition from animal to human clinical studies. In preferred embodiments of the invention, conserved oligonucleotides are used; this allows a single oligonucleotide sequence to be used in both animal models and human clinical trials.

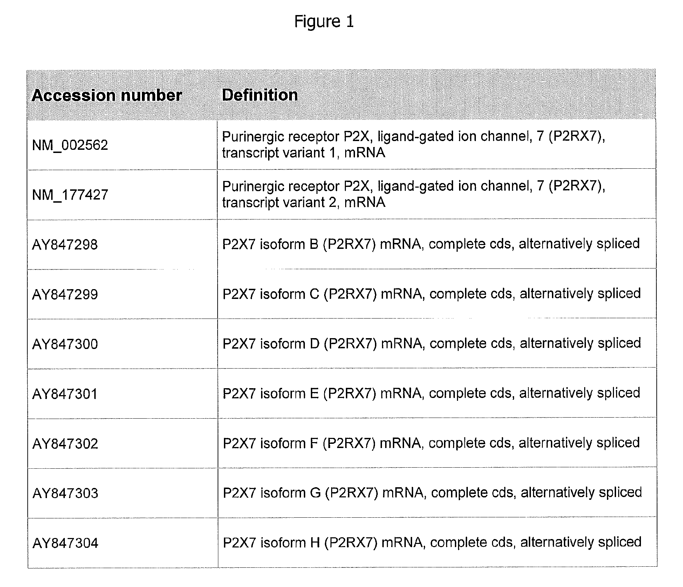

GenBank Accession Numbers corresponding to P2RX7 transcripts produced by alternative splicing are displayed in Figure 1.

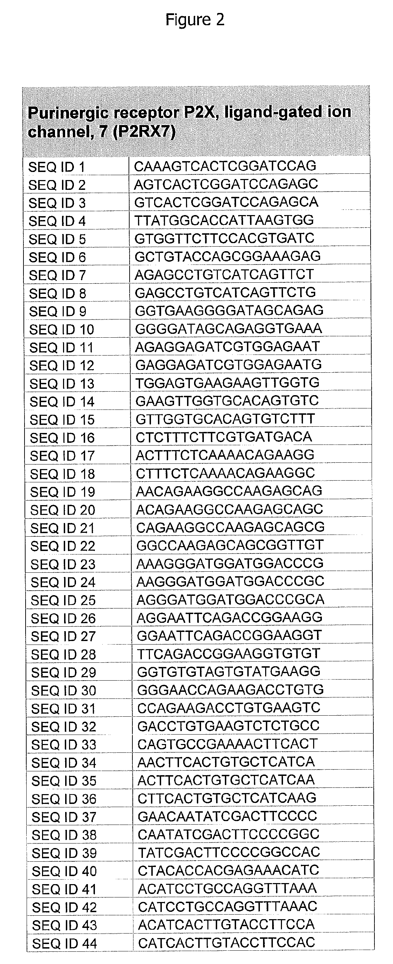

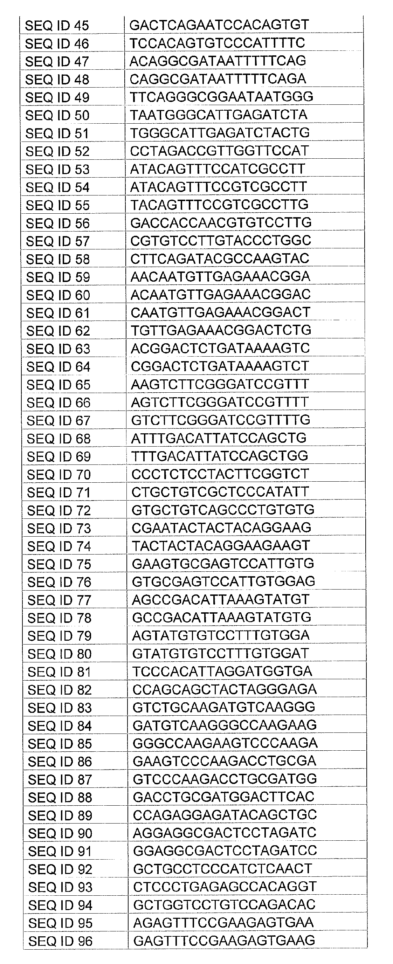

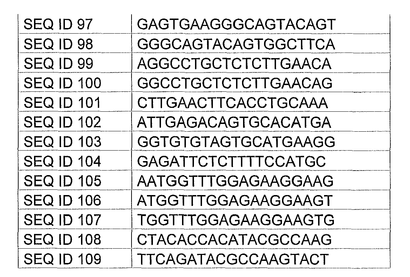

Selected oligonucleotide sequences against which RNAi is directed are shown in Figure 2. Displayed sequences are the DNA sequences targeted by the siNA. Therefore, the invention would make use of NA duplexes with sequences complementary to the indicated DNA sequences.

The sequences displayed in Figure 2 are not limiting. As a matter of fact, target DNA need not necessarily be preceded by AA or CA. Further, target DNA could be constituted by sequences included in Figure 2 flanked by any contiguous sequence.

### In vitro studies.

### Obtaining siRNA duplexes

RNAs are preferably chemically synthesized using appropriately protected ribonucleoside phosphoramidites and a conventional DNA/RNA synthesizer. Substitution of one or both strands of a siRNA duplex by 2'-deoxy or 2'-O-methyl oligoribonucleotides abolished silencing in fly extract (Elbashir *et al.* 2001). In mammalian cells, however, it seems possible to substitute the sense siRNA by a 2'-O-methyl oligoribonucleotide (Ge *et al.* 2003).

Most conveniently, siRNAs are obtained from commercial RNA oligo synthesis suppliers, which sell RNA-synthesis products of different quality and costs. In general, 21-nt RNAs are not too difficult to synthesize and are readily provided in a quality suitable for RNAi.

Suppliers of RNA synthesis reagents include Proligo (Hamburg, Germany), Dharmacon Research (Lafayette, CO, USA), Glen Research (Sterling, VA, USA), ChemGenes (Ashland, MA, USA), and Cruachem (Glasgow, UK), Qiagen (Germany), Ambion (USA) and Invitrogen (Scotland). The previous custom RNA synthesis companies are entitled to provide siRNAs with a license for target validation. In particular, our siRNA suppliers are Ambion, Dharmacon and Invitrogen, companies that offer the traditional custom chemical synthesis service for siRNA, and supply the siRNA with HPLC purification and delivered in dry form along with RNase-free water. A central web-based resource for RNAi and siRNA methodologies, along with links to additional siRNA related products and services, can be found on the website of above-mentioned suppliers.

An annealing step is necessary when working with single-stranded RNA molecules. It is critical that all handling steps be conducted under sterile, Rnase free conditions. To anneal the RNAs, the oligos must first be quantified by UV absorption at 260 nanometres (nm). The following protocol based on Elbashir et al. (2001) is then used for annealing:
- Separately aliquot and dilute each RNA oligo to a concentration of 50 µM.
- Combine 30 µl of each RNA oligo solution and 15 µl of 5X annealing buffer. Final buffer concentration is: 100 mM potassium acetate, 30 mM HEPES-KOH pH 7.4, 2 mM magnesium acetate. Final volume is 75 µl.
- Incubate the solution for 1 minute at 90 °C, centrifuge the tube for 15 seconds, let sit for 1 hour at 37 °C, then use at ambient temperature. The solution can be stored frozen at -20 °C and freeze-thawed up to 5 times. The final concentration of siRNA duplex is usually 20 µM.
Alternatively, already annealed dsRNAs may be purchased from the suppliers.

Chemically modified nucleic acids may also be used. For example, an overview of the types of modification which may be used is given in WO03/070744, the contents of which are incorporated herein by reference. Particular attention is drawn to pages 11 to 21 of this publication. Other possible modifications are as described above. The skilled person will be aware of other types of chemical modification which may be incorporated into RNA molecules.

### "In vitro" system

To check the specificity of the siRNA interference cell cultures and organotypic cultures both expressing the target gene, were employed.

The cells used for these experiments were murine muscle cells, C2C12, and the organotypic cultures were spinal cord slices. The levels of P2RX7 expression were analyzed after being incubated with the corresponding siRNA duplexes. For linking siRNA knockdown to specific phenotypes in cultured cells, it is necessary to demonstrate the decrease of the targeted protein or at least to demonstrate the reduction of the targeted mRNA.

mRNA levels of the target gene can be quantitated by Real-time quantitative PCR (qRT-PCR). Further, the protein levels can be determined in a variety of ways well known in the art, such as Western blot analysis with specific antibodies to the different target allow direct monitoring of the reduction of targeted protein.

### Transfection of siRNA duplexes in cell cultures.

Several examples of techniques well known in the art are as follows: We can perform a single transfection of siRNA duplex using a cationic lipid, such as Lipofectamine 2000 Reagent (Invitrogen) and assay for silencing 24, 48 and 72 hours after transfection.

A typical transfection protocol can be performed as follows: For one well of a 6-well plate, we transfect using 100 or 200nM as final concentration of siRNA. Following Lipofectamine 2000 Reagent protocol, the day before transfection, we seed 2-4 x 10⁵ cells per well in 3ml of an appropriate growth medium, containing DMEM, 10% serum, antibiotics and glutamine, and incubate cells under normal growth conditions (37°C and 5% CO₂). On the day of transfection, cells have to be at 30-50% confluence. We dilute 12.5ul of 20uM siRNA duplex (corresponding to 100 nM final concentration) or 25ul of 20uM siRNA duplex (corresponding to 200nM final concentration) in 250ul of DMEM and mix. Also, 6ul of Lipofectamine 2000 is diluted in 250ul of DMEM and mixed. After a 5 minutes incubation at room temperature, the diluted oligomer (siRNA duplex) and the diluted Lipofectamine are combined to allow complex formation during a 20 minutes incubation at room temperature. Afterwards, we add the complexes drop-wise onto the cells with 2 ml of fresh growth medium low in antibiotics and mix gently by rocking the plate back and forth, to ensure uniform distribution of the transfection complexes. We incubate the cells under their normal growth conditions and the day after the complexes are removed and fresh and complete growth medium is added. To monitor gene silencing cells are collected at 24, 48 and 72h post-transfection.

The efficiency of transfection may depend on the cell type, but also on the passage number and the confluency of the cells. The time and the manner of formation of siRNA-liposome complexes (e.g. inversion versus vortexing) are also critical. Low transfection efficiencies are the most frequent cause of unsuccessful silencing. Good transfection is a non-trivial issue and needs to be carefully examined for each new cell line to be used. Transfection efficiency may be tested transfecting reporter genes, for example a CMV-driven EGFP-expression plasmid (e.g. from Clontech) or a B-Gal expression plasmid, and then assessed by phase contrast and/or fluorescence microscopy the next day.

### Testing of siRNA duplexes

Depending on the abundance and the life time (or turnover) of the targeted protein, a knock-down phenotype may become apparent after 1 to 3 days, or even later. In cases where no phenotype is observed, depletion of the protein may be observed by immunofluorescence or Western blotting.

After transfections, total RNA fractions extracted from cells were pre-treated with DNase I and used for reverse transcription using a random primer. PCR-amplified with a specific primer pair covering at least one exon-exon junction in order to control for amplification of pre-mRNAs. RT/PCR of a non-targeted mRNA is also needed as control. Effective depletion of the mRNA yet undetectable reduction of target protein may indicate that a large reservoir of stable protein may exist in the cell. Alternatively, Real-time PCR amplification can be used to test in a more precise way the mRNA decrease or disappearance. Real-time reverse-transcriptase (RT) PCR quantitates the initial amount of the template most specifically, sensitively and reproducibly. Real-time PCR monitors the fluorescence emitted during the reaction as an indicator of amplicon production during each PCR cycle, in a light cycler apparatus. This signal increases in direct proportion to the amount of PCR product in a reaction. By recording the amount of fluorescence emission at each cycle, it is possible to monitor the PCR reaction during exponential phase where the first significant increase in the amount of PCR product correlates to the initial amount of target template.

To verify the interference pattern of the differentially expressed P2RX7 gene in the cell cultures, qRT-PCR was performed according to the manufacturer protocol (Roche). For quantitative qRT-PCR, approximately 500 ng of total RNA were used for reverse transcription followed by PCR amplification with specific primers for each gene in reaction mixture containing Master SYBR Green I. The PCR conditions were an initial step of 30 min at 91°C, followed by 40 cycles of 5 s at 95°C, 10 s at 62°C and 15 s at 72°C. Quantification of b-actin mRNA was used as a control for data normalization. Relative gene expression comparisons work best when the gene expression of the chosen endogenous/internal control is more abundant and remains constant, in proportion to total RNA, among the samples. By using an invariant endogenous control as an active reference, quantitation of an mRNA target can be normalised for differences in the amount of total RNA added to each reaction. The amplification curves obtained with the light cycler were analyzed in combination with the control kit RNA, which targets in vitro transcribed cytokine RNA template, according to the manufacturer protocol. In order to assess the specificity of the amplified PCR product a melting curve analysis was performed. The resulting melting curves allow discrimination between primer-dimers and specific PCR product.

### Transfection of siRNA duplexes in organotypic cultures

To obtain spinal cord organotypic cultures, the experimental protocol was performed as follows: The spinal cord was extracted from 6 to 8 week old rats and placed in ice-cold dissecting media containing Gey's Medium supplemented with D-Glucose (6.5 mg/ml) and 15mM Hepes. To generate the organotypic cultures, 500 µm slices from the thoracic spinal cord were obtained using a tissue chopper and placed in sterile MEM supplemented with Earl's salt solution. Spinal slices were transferred onto Millicell culture plates. Each plate, containing 4 to 6 slices, was placed into wells of a six-well plate containing 1.25ml of antibiotic-free medium (50% MEM with Earl's salts and glutamine, 25% Hanks balanced salt solution and 25% Horse Serum supplemented with D-Glucose (6mg/ml) and 20mM Hepes).

Slices were incubated under normal growth conditions (37°C and 5% CO₂) and media was changed the day after and, afterwards, three times a week.

In these conditions, spinal cord organotypic cultures maintain their structural integrity for at least 15 days and present high levels of the P2RX7 transcript. Also, P2RX7 gene expression, checked by quantitative PCR assays, does not present any relevant change along the culture period.

To perform siRNA transfections there are several protocols and techniques well known in the art. In this case a double transfection is needed to observe an enhanced gene expression inhibition. Double siRNA transfections were performed using a cationic lipid, such as Lipofectamine 2000 Reagent (Invitrogen) and gene expression silencing was assayed at different time points.

A typical transfection protocol can be performed as follows. Each Millicell culture plate, containing 4 to 6 slices, is transfected using a determined concentration of siRNA. Following Lipofectamine 2000 Reagent protocol for siRNA transfection, we dilute the amount of siRNA duplex in 50ul of MEM and mix. In a different tube, the Lipofectamine 2000 are diluted in 50ul of MEM and mixed. After 5 minutes incubation at room temperature, the diluted siRNA and the diluted Lipofectamine are combined to allow complex formation during 20 minutes incubation at room temperature. Afterwards, the complexes are added drop-wise over the slices. We incubate the slices under their normal growth conditions and the day after, the complexes are removed and fresh and complete growth medium is added. When necessary, 48h after the first Lipofectamine treatment the protocol is repeated as previously described.

The efficiency of transfection may depend on the cell or tissue type, but also on their culture characteristics. The time and the manner of formation of siRNA-liposome complexes are also critical. Low transfection efficiencies are the most frequent cause of unsuccessful silencing. Good transfection is a non-trivial issue and needs to be carefully examined for each new cell line to be used. Transfection efficiency may be tested transfecting reporter genes, for example a CMV-driven EGFP-expression plasmid (e.g. from Clontech) or a β-Gal expression plasmid, and then assessed by phase contrast and/or fluorescence microscopy the next day. Spinal cord organotypic cultures were successfully transfected with a β-Gal encoded construct reporter. The enzymatic activity of bacterial. β-Gal can be assayed readily from transfected tissue with an appropriate commercial staining set.

### Pharmaceutical formulations

The present invention may comprise the administration of one or more species of siNA molecule simultaneously. These species may be selected to target one or more target genes.

The siNA molecules of the invention and formulations or compositions thereof may be administered directly or topically (e. g., locally) to the organ of interest (for example, spinal cord, brain, etc) as is generally known in the art. For example, a siNA molecule can comprise a delivery vehicle, including liposomes, for administration to a subject. Carriers and diluents and their salts can be present in pharmaceutically acceptable formulations. Nucleic acid molecules can be administered to cells by a variety of methods known to those of skill in the art, including, but not restricted to, encapsulation in liposomes, by iontophoresis, or by incorporation into other vehicles, such as biodegradable polymers, hydrogels, cyclodextrins poly (lactic-co-glycolic) acid (PLGA) and PLCA microspheres, biodegradable nanocapsules, and bioadhesive microspheres, or by proteinaceous vectors. In another embodiment, the nucleic acid molecules of the invention can also be formulated or complexed with polyethyleneimine and derivatives thereof, such as polyethyleneimine-polyethyleneglycol-N-acetylgalactosamine (PEI-PEG-GAL) or polyethyleneimine-polyethyleneglycol-tri-N-acetylgalactosamine (PEI-PEG-triGAL) derivatives.

A siNA molecule of the invention may be complexed with membrane disruptive agents and/or a cationic lipid or helper lipid molecule.

Delivery systems which may be used with the invention include, for example, aqueous and non aqueous gels, creams, multiple emulsions, microemulsions, liposomes, ointments, aqueous and non aqueous solutions, lotions, aerosols, hydrocarbon bases and powders, and can contain excipients such as solubilizers, permeation enhancers (e. g., fatty acids, fatty acid esters, fatty alcohols and amino acids), and hydrophilic polymers (e. g. , polycarbophil and polyvinylpyrolidone). In one embodiment, the pharmaceutically acceptable carrier is a liposome or a transdermal enhancer.

A pharmaceutical formulation of the invention is in a form suitable for administration, e.g., systemic or local administration, into a cell or subject, including for example a human. Suitable forms, in part, depend upon the use or the route of entry, for example oral, transdermal, or by injection. Other factors are known in the art, and include considerations such as toxicity and forms that prevent the composition or formulation from exerting its effect.

The present invention also includes compositions prepared for storage or administration that include a pharmaceutically effective amount of the desired compounds in a pharmaceutically acceptable carrier or diluent. Acceptable carriers or diluents for therapeutic use are well known in the pharmaceutical art. For example, preservatives, stabilizers, dyes and flavouring agents can be provided. These include sodium benzoate, sorbic acid and esters of p-hydroxybenzoic acid. In addition, antioxidants and suspending agents can be used.

A pharmaceutically effective dose is that dose required to prevent, inhibit the occurrence, or treat (alleviate a symptom to some extent, preferably all of the symptoms) of a disease state. The pharmaceutically effective dose depends on the type of disease, the composition used, the route of administration, the type of mammal being treated, the physical characteristics of the specific mammal under consideration, concurrent medication, and other factors that those skilled in the medical arts will recognize.

Generally, an amount between 0.1mg/kg and 100 mg/kg body weight/day of active ingredients is administered.

The formulations of the invention can be administered in unit dosage formulations containing conventional non-toxic pharmaceutically acceptable carriers, adjuvants and/or vehicles. Formulations can be in a form suitable for oral use, for example, as tablets, troches, lozenges, aqueous or oily suspensions, dispersible powders or granules, emulsion, hard or soft capsules, or syrups or elixirs. Compositions intended for oral use can be prepared according to any method known to the art for the manufacture of pharmaceutical compositions and such compositions can contain one or more such sweetening agents, flavouring agents, colouring agents or preservative agents in order to provide pharmaceutically elegant and palatable preparations. Tablets contain the active ingredient in admixture with non-toxic pharmaceutically acceptable excipients that are suitable for the manufacture of tablets.

These excipients can be, for example, inert diluents; such as calcium carbonate, sodium carbonate, lactose, calcium phosphate or sodium phosphate; granulating and disintegrating agents, for example, corn starch, or alginic acid; binding agents, for example starch, gelatin or acacia; and lubricating agents, for example magnesium stearate, stearic acid or talc. The tablets can be uncoated or they can be coated by known techniques. In some cases such coatings can be prepared by known techniques to delay disintegration and absorption in the gastrointestinal tract and thereby provide a sustained action over a longer period. For example, a time delay material such as glyceryl monostearate or glyceryl distearate can be employed.

Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in a mixture with excipients suitable for the manufacture of aqueous suspensions. Such excipients are suspending agents, for example sodium carboxymethylcellulose, methylcellulose, hydropropyl- methylcellulose, sodium alginate, polyvinylpyrrolidone, gum tragacanth and gum acacia; dispersing or wetting agents can be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions can also contain one or more preservatives, for example ethyl, or n-propyl p-hydroxybenzoate, one or more colouring agents, one or more flavouring agents, and one or more sweetening agents, such as sucrose or saccharin.

Oily suspensions can be formulated by suspending the active ingredients in a vegetable oil, for example arachis oil, olive oil, sesame oil or coconut oil, or in a mineral oil such as liquid paraffin. The oily suspensions can contain a thickening agent, for example beeswax, hard paraffin or cetyl alcohol. Sweetening agents and flavouring agents can be added to provide palatable oral preparations. These compositions can be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents or suspending agents are exemplified by those already mentioned above. Additional excipients, for example sweetening, flavouring and colouring agents, can also be present.

Pharmaceutical compositions of the invention can also be in the form of oil-in-water emulsions. The oily phase can be a vegetable oil or a mineral oil or mixtures of these. Suitable emulsifying agents can be naturally-occurring gums, for example gum acacia or gum tragacanth, naturally-occurring phosphatides, for example soy bean, lecithin, and esters or partial esters derived from fatty acids and hexitol, anhydrides, for example sorbitan monooleate, and condensation products of the said partial esters with ethylene oxide, for example polyoxyethylene sorbitan monooleate. The emulsions can also contain sweetening and flavouring agents.

Syrups and elixirs can be formulated with sweetening agents, for example glycerol, propylene glycol, sorbitol, glucose or sucrose. Such formulations can also contain a demulcent, a preservative and flavouring and colouring agents. The pharmaceutical compositions can be in the form of a sterile injectable aqueous or oleaginous suspension.

This suspension can be formulated according to the known art using those suitable dispersing or wetting agents and suspending agents that have been mentioned above.

A sterile injectable preparation can also be a sterile injectable solution or suspension in a non-toxic parentally acceptable diluent or solvent, for example as a solution in 1,3- butanediol. Among the acceptable vehicles and solvents that can be employed are water, Ringer's solution and isotonic sodium chloride solution. In addition, sterile, fixed oils are conventionally employed as a solvent or suspending medium. For this purpose, any bland fixed oil can be employed including synthetic mono-or diglycerides. In addition, fatty acids such as oleic acid find use in the preparation of injectables.

The nucleic acid molecules of the invention can also be administered in the form of suppositories, e. g. , for rectal administration of the drug. These compositions can be prepared by mixing the drug with a suitable non-irritating excipient that is solid at ordinary temperatures but liquid at the rectal temperature and will therefore melt in the rectum to release the drug. Such materials include cocoa butter and polyethylene glycols.

Nucleic acid molecules of the invention can be administered parenterally in a sterile medium. The drug, depending on the vehicle and concentration used, can either be suspended or dissolved in the vehicle. Advantageously, adjuvants such as local anaesthetics, preservatives and buffering agents can be dissolved in the vehicle.

It is understood that the specific dose level for any particular subject depends upon a variety of factors including the activity of the specific compound employed, the age, body weight, general health, sex, diet, time of administration, route of administration, and rate of excretion, drug combination and the severity of the particular disease undergoing therapy.

For administration to non-human animals, the composition can also be added to the animal feed or drinking water. It can be convenient to formulate the animal feed and drinking water compositions so that the animal takes in a therapeutically appropriate quantity of the composition along with its diet. It can also be convenient to present the composition as a premix for addition to the feed or drinking water.

The nucleic acid molecules of the present invention can also be administered to a subject in combination with other therapeutic compounds to increase the overall therapeutic effect. The use of multiple compounds to treat an indication can increase the beneficial effects while reducing the presence of side effects.

Alternatively, certain siNA molecules of the invention can be expressed within cells from eukaryotic promoters. Recombinant vectors capable of expressing the siNA molecules can be delivered and persist in target cells. Alternatively, vectors can be used that provide for transient expression of nucleic acid molecules. Such vectors can be repeatedly administered as necessary. Once expressed, the siNA molecule interacts with the target mRNA and generates an RNAi response. Delivery of siNA molecule expressing vectors can be systemic, such as by intravenous or intra-muscular administration, by administration to target cells ex-planted from a subject followed by reintroduction into the subject, or by any other means that would allow for introduction into the desired target cell.

### Results

### Example 1. In vitro assays.

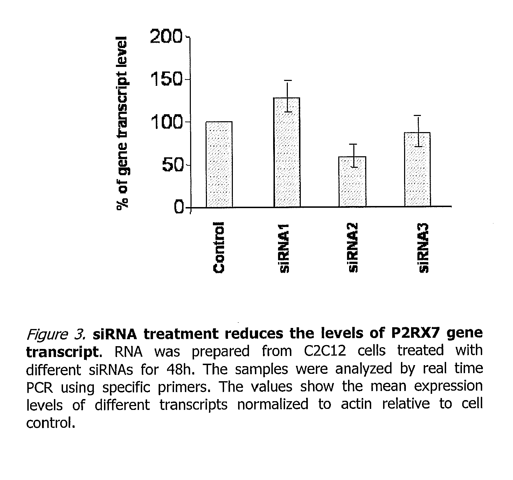

A panel of siRNA against the P2RX7 target gene has been analyzed. The first step was to perform experiments in cell cultures. For the P2RX7 target gene, several siRNAs were designed using a specific software according to the rules described before. Those with the best characteristics were selected to be tested. The siRNAs were applied to cell cultures, such as C2C12. The effect of siRNAs over the target gene was analyzed by Real-time PCR according to the manufacturer's protocol. The gene target transcript levels were normalized using actin as housekeeping gene. Some of the different siRNAs that were tested and their different efficacies in the interference of the target gene are included in Figure 3. RNA was prepared from C2C12 cells treated with different siRNAs for 48h. The samples were analyzed by real time PCR using specific primers. The values show the mean expression levels of different transcripts normalized to actin relative to cell control. siRNA1, siRNA2 and siRNA3 target the murine sequences homologous to the human sequences listed in Figure 2 as follows: siRNA1 targets the murine sequence homologous to human SEQ. ID. 37; siRNA2 targets the murine sequence homologous to human SEQ. ID. 78; and siRNA3 targets the murine sequence homologous to human SEQ. ID. 92. The values represent the mean of the percentage of the normalized mRNA levels upon siRNA interference over the control gene expression and their standard deviations. The level of the P2RX7 transcript after the siRNA treatment was highly reduced with siRNA2 and siRNA3, compared to the control cells. The decrease of the gene expression depends on the efficiency in siRNA silencing. In fact, siRNA2 treatment decreased the P2RX7 gene expression to 58 % compared to the control.

### Example 2. Time-dose response in vitro.

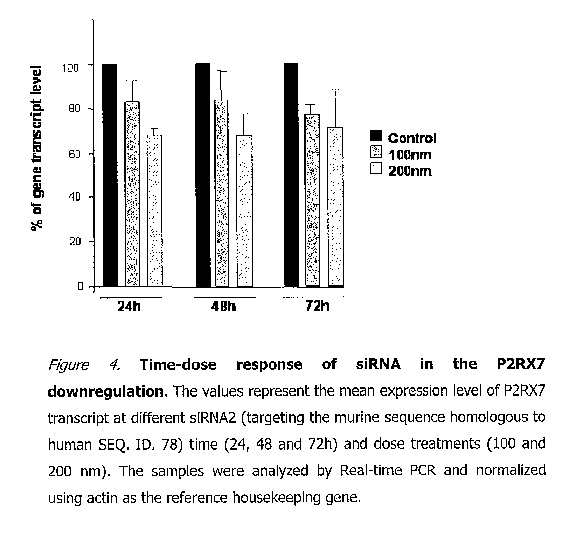

In order to validate the efficiency of siRNA2, more treatments were carried out in C2C12 cells. Cells were transfected with siRNA2 and the level of P2RX7 transcript was analyzed by Real-time PCR at 24, 48 and 72 h. The level of the transcript was significantly reduced at this time points after the siRNA treatment. Figure 4 shows the mean of the percentage of the normalized mRNA P2RX7 levels upon siRNA interference over the control gene expression at each time point and their standard deviations. Moreover a siRNA dose-response was analyzed. Figure 4 shows the results of two different siRNA applications (100 and 200nm). 200nm siRNA applications were more effective in the P2RX7 downregulation than those with 100nm, confirming both the specificity and the effectiveness of the treatment.

### Example 3. Organotypic cultures.

Previously to the siRNA spinal cord application, we performed in vitro experiments using an established model based on spinal cord slice cultures. It is essential to use appropriate experimental models in order to understand the complex processes which evolve after the initial trauma. This model facilitated the investigation of primary and secondary mechanisms of cell death that occurs after spinal cord injury and represents a step before study of the effect of P2RX7 interference in murine models is undertaken. Several in vivo models have been characterized to study the chronic pathology. However, due to the complexity of the in vivo system, interpretation of results may be more difficult, plus the cost of maintaining an animal model is very high. In order to study the events after trauma the in vitro models are preferred, as these allow precise control over the environment, and easy and repeated access.

Previously to the experiment, the morphological integrity of cultures was examined by microscopy and the delivery in this model was carried out transfecting a reporter gene (β-gal) according to the described protocol. Upon transfecting cells with a β-gal construct, spinal cords were fixed, washed and stained with freshly prepared X-gal staining solution. Blue staining appeared upon 24 h indicating a successfully delivery.

Two siRNAs were selected according to different criteria, siRNAH and siRNAR. siRNAH targets the rat sequence homologous to human SEQ. ID. 58 of Figure 2, while siRNAR targets the best candidate sequence in rat, selected by specific software, homologous to human SEQ. ID. 37 of Figure 2. Spinal cord cultures were obtained as previously described. Each plate was double transfected with the corresponding siRNA and gene expression was assayed at 72 and 96 h after the first transfection by Real- time PCR.

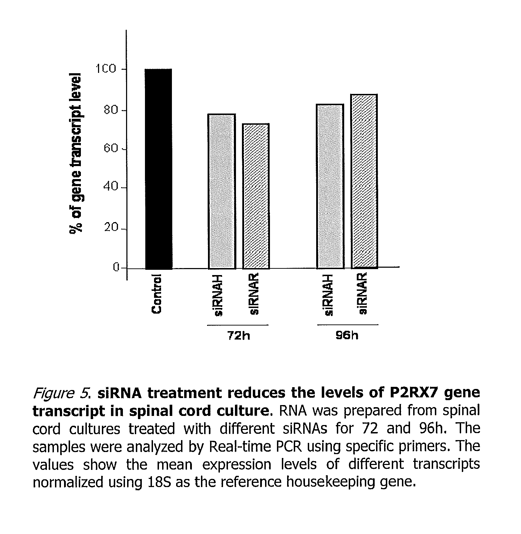

Figure 5 shows a representative experiment. Values represent the percentage of mRNA transcript relative to the control after siRNA treatment once normalized using 18S as the reference housekeeping gene. Previous experiments indicated 18S as a better reference than β actin in spinal cord cultures analysis. The decrease in P2RX7 transcript is higher at 72 h being around 70%, at 96h there is less reduction, around 80-90%. This set of experiments confirms the ability of RNAi to reduce P2RX7 expression in an organotypic culture very close to an in vivo model.

### REFERENCES

Akashi H, Miyagishi M, Taira K. Suppression of gene expression by RNA interference in cultured plant cells. Antisense Nucleic Acid Drug Dev, 2001, 11(6):359-67.
Banerjee D, Slack F. Control of developmental timing by small temporal RNAs: a paradigm for RNA-mediated regulation of gene expression. Bioessays, 2002, 24(2):119-29.
Berchtold, S., Ogilvie, A. L. J., Bogdan, C., Muhl-Zurbes, P., Ogilvie, A., Schuler, G. and Steinkasserer, A. Human monocyte derived dendritic cells express functional P2X and P2Y receptors as well as ecto-nucleotidases. FEBS Lett., 1999, 458:424.
Bonnefy, J.-Y., Plater-Zyberk, C., Lecoanet-Henchoz, S., Gauchat, J.-F., Aubry, J.-P. and Graber, P. A new role for CD23 in infammation. Immunol. Today, 1996, 17:418.
Bosher JM, Labouesse M. RNA interference: genetic wand and genetic watchdog. Nat Cell Biol, 2000, 2(2):E31-6.
Braasch DA, Corey DR. Novel antisense and peptide nucleic acid strategies for controlling gene expression. Biochemistry, 2002, 41(14):4503-10.
Budagian V, Bulanova E, Brovko L, Orinska Z, Fayad R, Paus R, Bulfone-Paus S. Signaling through P2X7 receptor in human T cells involves p56lck, MAP kinases, and transcription factors AP-1 and NF-kappa B. J Biol Chem., 2003 Jan 17; 278(3): 1549-60.
Cabrini G, Falzoni S, Forchap SL, Pellegatti P, Balboni A, Agostini P, Cuneo A, Castoldi G, Baricordi OR, Di Virgilio F. A His-155 to Tyr polymorphism confers gain-of-function to the human P2X7 receptor of human leukemic lymphocytes. J Immunol., 2005 Jul 1;175(1):82-9.
Caplen, N.J., Parrish, S., Imani, F., Fire, A. & Morgan, R.A. Specific inhibition of gene expression by small double stranded RNAs in invertebrate and vertebrate systems. Proc. Natl. Acad. Sci. USA, 2001, 98: 9742-9747.
Cheewatrakoolpong B, Gilchrest H, Anthes JC, Greenfeder S. Identification and characterization of splice variants of the human P2X7 ATP channel. Biochem Biophys Res Commun., 2005 Jun 24;332(1):17-27.
Chessell IP, Hatcher JP, Bountra C, Michel AD, Hughes JP, Green P, Egerton J, Murfin M, Richardson J, Peck WL, Grahames CB, Casula MA, Yiangou Y, Birch R, Anand P, Buell GN. Disruption of the P2X7 purinoceptor gene abolishes chronic inflammatory and neuropathic pain. Pain, 2005 Apr;114(3):386-96.
Cook, S.P. & McCleskey, E.W. Cell damage excites nociceptors through release of cytosolic ATP. Pain, 2002, 95: 41-47.
Cotrina, M.L. et al. Connexins regulate calcium signalling by controlling ATP release. Proc. Natl. Acad. Sci. USA, 1998,.95: 15735-15740.
Deuchars, S.A. et al. Neuronal P2X7 receptors are targeted to presynaptic terminals in the central and peripheral nervous systems. J. Neurosci., 2001, 21: 7143-7152.
Di Virgilio, F., Chiozzi, P., Ferrari, D., Falzoni, S., Sanz, J. M., Morelli, A., Torboli, M., Bolognesi, G., and Baricordi, O. R. Nucleotide receptors: an emerging family of regulatory molecules in blood cells. Blood, 2001, 97, 587-600.
Di Virgilio, F. et al. Cytolytic P2X purinoceptors. Cell Death Differ., 1998, 5: 191-199.
Du, S. et al. Calcium influx and activation of calpain I mediate acute reactive gliosis in injured spinal cord. Exp. Neurol., 1999, 157: 96-105.
El-Moatassim, C., and Dubyak, G. R. Dissociation of the pore-forming and phospholipase D activities stimulated via P2z purinergic receptors in BAC1.2F5 macrophages. Product inhibition of phospholipase D enzyme activity J. Biol. Chem., 1993, 268, 15571-15578.
Elbashir SM, Lendeckel W, Tuschl T. RNA interference is mediated by 21- and 22-nucleotide RNAs. Genes Dev., 2001, 15(2):188-200.
Fam, S.R., Gallagher, C.J. & Salter, M.W. P2Y(1) purinoceptor-mediated Ca2+ signalling and Ca2+ wave propagation in dorsal spinal cord astrocytes. J. Neurosci., 2000, 20: 2800-2808.
Ferrari, D., Los, M., Bauer, M. K. A., Vandenabeele, P., Wesselborg, S., and Schulze-Osthoff, K. P2Z purinoreceptor ligation induces activation of caspases with distinct roles in apoptotic and necrotic alterations of cell death. FEBS Lett., 1999, 447, 71-75.
Ferrari, D., La Sala, A., Chiozzi, P., Morelli, A., Falzoni, S., Girolomoni, G., Idzko, M., Dichmann, S., Norgauer, J. and Di Virgilio, F. The P2 purinergic receptors of human dendritic cells: identi®cation and coupling to cytokine release. FASEB J., 2000, 14:2466.
Fields, R.D. & Stevens-Graham, B. New insights into neuron-glia communication. Science, 2002, 298: 556-562.
Fire A, Xu S, Montgomery MK, Kostas SA, Driver SE, Mello CC. Potent and specific genetic interference by double stranded RNA in Caenorhabditis elegans. Nature, 1998, 391(6669):806-11.
Franke H, Gunther A, Grosche J, Schmidt R, Rossner S, Reinhardt R, Faber-Zuschratter H, Schneider D, Illes P. P2X7 receptor expression after ischemia in the cerebral cortex of rats. J Neuropathol Exp Neurol., 2004 Jul; 63(7):686-99. Gargett, C. E., Cornish, E. J., and Wiley, J. S. Phospholipase D activation by P2Z-purinoceptor agonists in human lymphocytes is dependent on bivalent cation influx. Biochem. J., 1996, 313, 529-535.
Ge Q, McManus MT, Nguyen T, Shen CH, Sharp PA, Eisen HN, Chen J. RNA interference of influenza virus production by directly targeting mRNA for degradation and indirectly inhibiting all viral RNA transcription. Proc Natl Acad Sci U S A., 2003; 100(5):2718-23.
Gil J, Esteban M. Induction of apoptosis by the dsRNA-dependent protein kinase (PKR): mechanism of action. Apoptosis, 2000, 5(2):107-14.
Greig AV, Linge C, Healy V, Lim P, Clayton E, Rustin MH, McGrouther DA, Burnstock G. Expression of purinergic receptors in non-melanoma skin cancers and their functional roles in A431 cells. J Invest Dermatol., 2003a, 121(2):315-27.
Greig AV, Linge C, Terenghi G, McGrouther DA, Burnstock G. Purinergic receptors are part of a functional signaling system for proliferation and differentiation of human epidermal keratinocytes. J Invest Dermatol., 2003b, 120(6):1007-15.
Grosshans H, Slack FJ. Micro-RNAs: small is plentiful. J Cell Biol, 2002, 156(1):17-21.
Gu, B., Bendall, L. J., and Wiley, J. S. Adenosine Triphosphate-Induced Shedding of CD23 and L-Selectin (CD62L) From Lymphocytes Is Mediated by the Same Receptor but Different Metalloproteases. Blood, 1998, 92, 946-951. Guthrie et al. ATP released from astrocytes mediates glial calcium waves. J. Neurosci., 1999, 19: 520-528.
Hart, D. N. J. Dendritic cells: unique leukocyte populations which control the primary immune response. Blood, 1997, 90:3245.
Humphreys, B. D., Rice, J., Kertesy, S. B., and Dubyak, G. R. Stress-activated Protein Kinase/JNK Activation and Apoptotic Induction by the Macrophage P2X7 Nucleotide Receptor. J. Biol. Chem., 2000, 275, 26792-26798.
Jamieson, G. P., Snook, M. B., Thurlow, P. J., and Wiley, J. S. Extracellular ATP causes of loss of L-selectin from human lymphocytes via occupancy of P2Z purinocepters. J. Cell. Physiol., 1996, 166, 637-642.
Kawamura H, Sugiyama T, Wu DM, Kobayashi M, Yamanishi S, Katsumura K, Puro DG. ATP: a vasoactive signal in the pericyte-containing microvasculature of the rat retina. J Physiol., 2003 Sep 15;551(Pt 3):787-99. Epub 2003 Jul 22. Mutini C, Falzoni S, Ferrari D, Chiozzi P, Morelli A, Baricordi OR, Collo G, Ricciardi-Castagnoli P, Di Virgilio F. Mouse dendritic cells express the P2X7 purinergic receptor: characterization and possible participation in antigen presentation. J Immunol., 1999 Aug 15;163(4):1958-65.
Neary, J.T. et al. Activation of extracellular signal-regulated kinase by stretch-induced injury in astrocytes involves extracellular ATP and P2 purinergic receptors. J. Neurosci., 2003, 23: 2348-2356.
North, R. A. Molecular Physiology of P2X Receptors. Physiol. Rev., 2002, 82, 1013-1067.
Paddison PJ, Caudy AA, Bernstein E, Hannon GJ, Conklin DS. Short hairpin RNAs (shRNAs) induce sequence-specific silencing in mammalian cells. Genes Dev., 2002, 16(8):948-58.
Pannicke T, Fischer W, Biedermann B, Schadlich H, Grosche J, Faude F, Wiedemann P, Allgaier C, Illes P, Burnstock G, Reichenbach A. P2X7 receptors in Muller glial cells from the human retina. J Neurosci., 2000 Aug 15; 20(16):5965-72.
Parvathenani LK, Tertyshnikova S, Greco CR, Roberts SB, Robertson B, Posmantur R. P2X7 mediates superoxide production in primary microglia and is up-regulated in a transgenic mouse model of Alzheimer's disease. J Biol Chem., 2003 Apr 11;278(15):13309-17.
Scemes, E., Suadicani, S.O. & Spray, D.C. Intercellular communication in spinal cord astrocytes: fine tuning between gap junctions and P2 nucleotide receptors in calcium wave propagation. J. Neurosci., 2000, 20: 1435-1445.
Sluyter, R., and Wiley, J. S. Extracellular adenosine 5'-triphosphate induces a loss of CD23 from human dendritic cells via activation of P2X7 receptors. Int. Immunol., 2002, 14, 1415-1421.
Solini A, Chiozzi P, Morelli A, Adinolfi E, Rizzo R, Baricordi OR, Di Virgilio F. Enhanced P2X7 activity in human fibroblasts from diabetic patients: a possible pathogenetic mechanism for vascular damage in diabetes. Arterioscler Thromb Vasc Biol., 2004 Jul;24(7):1240-5.
Stockwin, L. H., McGonagle, D., Martin, I. G. and Blair, G. E. Dendritic cells: immunological sentinels with a central role in health and disease. Immunol. Cell Biol., 2000, 78:91.
Taylor AL, Schwiebert LM, Smith JJ, King C, Jones JR, Sorscher EJ, Schwiebert EM. Epithelial P2X purinergic receptor channel expression and function. J Clin Invest., 1999 Oct;104(7):875-84.
Tuschl T, Zamore PD, Lehmann R, Bartel DP, Sharp PA. Targeted mRNA degradation by double-stranded RNA in vitro. Genes Dev., 1999; 13(24):3191-7.
Verhoef PA, Estacion M, Schilling W, Dubyak GR. P2X7 receptor-dependent blebbing and the activation of Rho-effector kinases, caspases, and IL-1 beta release. J Immunol., 2003 Jun 1;170(11):5728-38.
Virginio, C., MacKenzie, A., North, R. A., and Surprenant, A. Kinetics of cell lysis, dye uptake and permeability changes in cells expressing the rat P2X7 receptor. J. Physiol. (Lond.), 1999, 519, 335-346.
Wang X, Arcuino G, Takano T, Lin J, Peng WG, Wan P, Li P, Xu Q, Liu QS, Goldman SA, Nedergaard M. P2X7 receptor inhibition improves recovery after spinal cord injury. Nat Med., 2004 Aug;10(8):821-7. Epub 2004 Jul 18.
Wianny F, Zernicka-Goetz M. Specific interference with gene function by double-stranded RNA in early mouse development. Nat Cell Biol., 2000, 2(2):70-5.
Williams BR. Role of the double-stranded RNA-activated protein kinase (PKR) in cell regulation. Biochem Soc Trans, 1997, 25(2):509-13.
Zhang X, Zhang M, Laties AM, Mitchell CH. Stimulation of P2X7 receptors elevates Ca2+ and kills retinal ganglion cells. Invest Ophthalmol Vis Sci., 2005 Jun; 46(6):2183-91.
Zhang XJ, Zheng GG, Ma XT, Yang YH, Li G, Rao Q, Nie K, Wu KF. Expression of P2X7 in human hematopoietic cell lines and leukemia patients. Leuk Res., 2004 Dec;28(12): 1313-22.

**We also describe herein the following further aspects:**
1. Use of siNA in the preparation of a medicament for use in a method of treatment of a condition characterised by increased expression and/or activity of P2RX7, the method comprising downregulating expression of P2RX7 in a patient.
2. The use of claim 1 wherein the condition is selected from the group comprising neuronal degeneration, reperfusion or ischemia in stroke or heart attack, Alzheimer's disease, inflammatory diseases (such as rheumatoid arthritis, osteoarthritis, asthma, rhinitis, chronic obstructive pulmonary disease (COPD), inflammatory bowel disease (IBD) such as Crohn's disease), allergies, autoimmune diseases, cancer (such as leukaemia or non-melanoma skin cancer), skin-related conditions (such as psoriasis, eczema, alopecia), retinal diseases and treatment of pain of neuropathic and inflammatory origin.
3. The use of any preceding claim wherein the siNA is siRNA.
4. The use of claim 3 wherein the siRNA is dsRNA.
5. The use of claim 3 wherein the siRNA is shRNA.
6. The use of any preceding claim wherein the siNA comprises a modified oligonucleotide.
7. The use of any preceding claim wherein the siNA is administered topically to a patient.
8. The use of any preceding claim wherein a plurality of species of siNA are used.
9. The use of claim 8 wherein said plurality of species are targeted to the same mRNA species.
10. The use of claim 8 wherein said plurality of species are targeted to different mRNA species.
11. The use of any preceding claim wherein the siNA is targeted to a sequence selected from SEQ ID 1 to SEQ ID 109.
12. The use of any preceding claim wherein the siNA is targeted to a splice form of P2RX7 selected from the group having GenBank Accession Numbers NM_002562, NM_177427, AY847298, AY847299, AY847300, AY847301, AY847302, AY847303, AY847304.
13. A method of treatment of a disease condition characterised by increased expression and/or activity of P2RX7, comprising administering siNA to downregulate expression of P2RX7 gene in a patient.
14. The method of claim 13, wherein the disease condition is selected from the group comprising neuronal degeneration, reperfusion or ischemia in stroke or heart attack, Alzheimer's disease, inflammatory diseases (such as rheumatoid arthritis, osteoarthritis, asthma, rhinitis, chronic obstructive pulmonary disease (COPD), inflammatory bowel disease (IBD) such as Crohn's disease), allergies, autoimmune diseases, cancer (such as leukaemia or non-melanoma skin cancer), skin-related conditions (such as psoriasis, eczema, alopecia), retinal diseases and treatment of pain of neuropathic and inflammatory origin.
15. The method of claim 13, wherein the disease condition is neuronal degeneration.
16. The method of claim 13, wherein the disease condition is reperfusion or ischemia in stroke or heart attack.
17. The method of claim 13, wherein the disease condition is Alzheimer's disease.
18. The method of claim 13, wherein the disease condition is an inflammatory disease (such as rheumatoid arthritis, osteoarthritis, asthma, rhinitis, chronic obstructive pulmonary disease (COPD), inflammatory bowel disease (IBD) such as Crohn's disease).
19. The method of claim 13, wherein the disease condition is an allergy.
20. The method of claim 13, wherein the disease condition is an autoimmune disease.
21. The method of claim 13, wherein the disease condition is cancer (such as leukaemia or non-melanoma skin cancer).
22. The method of claim 13, wherein the disease condition is a skin-related condition (such as psoriasis, eczema, alopecia).
23. The method of claim 13, wherein the disease condition is a retinal disease.
24. The method of claim 13, wherein the disease condition is pain of neuropathic and inflammatory origin.
25. The method of claim 13 wherein the siNA is siRNA.
26. The method of claim 25 wherein the siNA is dsRNA.
27. The method of claim 25 wherein the siNA is shRNA.
28. The method of claim 13 wherein the siNA comprises a modified oligonucleotide.
29. An isolated siNA molecule for use in the treatment of a disease condition characterized by increased expression and/or activity of P2RX7, the siNA being complementary to a nucleotide sequence selected from SEQ ID 1 to SEQ ID 109.
30. Use of an isolated siNA molecule having a sequence which is complementary to a nucleotide sequence selected from SEQ ID 1 to SEQ ID 109 in the preparation of a medicament for the treatment of a disease condition.
31. A pharmaceutical composition comprising siNA having a sequence which is complementary to a nucleotide sequence selected from SEQ ID 1 to SEQ ID 109.
32. Method for the treatment of a condition associated with, or mediated by, an increase of extra cellular calcium, comprising downregulation of P2RX7 gene expression by administering to a subject a pharmaceutically effective preparation for causing RNAi.
33. The method of claim 32, in which the condition is associated with reduced blood flow to the brain and other CNS tissue, or associated with instances of a temporary break in blood supply to the brain or to other CNS tissue.
34. The method of claim 32 or 33, in which the condition is an ischaemic disease, an anoxic episode, an injury to the brain and other parts of the CNS caused by trauma or other injury, a blow to the head, or a spinal injury, a thromboembolic or haemorrhagic stroke, a cerebral vasospasm, hypoglycaemia, cardiac arrest, status epilepticus, perinatal asphyxia, anoxia, cerebral trauma, lathyrism, Alzheimer's disease, Parkinson's Disease and Huntington's disease, cerebral ischaemia or cerebral infarction, ischaemic, hypoxic or anoxic brain damage, spinal cord injury, tissue ischaemia and reperfusion injury in a mammal at risk for such damage.
35. A method for the detection of a pharmacological activity in a biological sample comprising administering a sample to a cell, and determining a change in activity of P2RX7.
36. The method of claim 35, wherein a change in activity is determined by comparing a cell in which P2RX7 gene expression has been downregulated with a normal cell to which the sample has been administered.
37. The method of claim 36 in which P2RX7 downregulation is effected using siNA.
38. The method of any of claims 35 to 37, wherein the biological sample is of marine origin.

## Claims

1. Use of siRNA targeted to SEQ ID NO: 78 in the preparation of a medicament for use in a method of treatment of a condition **characterised by** increased expression and/or activity of P2RX7, the method comprising downregulating expression of P2RX7 in a patient.

2. The use of claim 1 wherein the condition is selected from the group comprising neuronal degeneration, reperfusion or ischemia in stroke or heart attack, Alzheimer's disease, inflammatory diseases (such as rheumatoid arthritis, osteoarthritis, asthma, rhinitis, chronic obstructive pulmonary disease (COPD), inflammatory bowel disease (IBD) such as Crohn's disease), allergies, autoimmune diseases, cancer (such as leukaemia or non-melanoma skin cancer), skin-related conditions (such as psoriasis, eczema, alopecia), retinal diseases and treatment of pain of neuropathic and inflammatory origin.

3. The use of claim 1 wherein the siRNA is dsRNA.

4. The use of claim 1 wherein the siRNA is shRNA.

5. The use of any preceding claim wherein the siRNA comprises a modified oligonucleotide.

6. The use of any preceding claim wherein the siRNA is administered topically to a patient.

7. The use of any preceding claim wherein a plurality of species of siRNA are used in combination with that of claim 1.

8. The use of claim 7 wherein said plurality of species are targeted to the same mRNA species.

9. The use of claim 7 wherein said plurality of species are targeted to different mRNA species.

10. The use of claim 8 wherein the plurality of species of siRNA are targeted to a sequence selected from SEQ ID 1 to SEQ ID 109.

11. An isolated siRNA molecule for use in the treatment of a disease condition **characterized by** increased expression and/or activity of P2RX7, the siRNA being complementary to a nucleotide sequence of SEQ ID 78.

12. A pharmaceutical composition comprising siNA having a sequence which is complementary to the nucleotide sequence of SEQ ID 78.

13. Use of an isolated siRNA molecule having a sequence which is complementary to a nucleotide sequence of SEQ ID 78 in the preparation of a medicament for the treatment of a disease condition.

14. The use of claim 13, in which the condition is associated with reduced blood flow to the brain and other CNS tissue, or associated with instances of a temporary break in blood supply to the brain or to other CNS tissue.

15. The use of claim 13 or 14, in which the condition is an ischaemic disease, an anoxic episode, an injury to the brain and other parts of the CNS caused by trauma or other injury, a blow to the head, or a spinal injury, a thromboembolic or haemorrhagic stroke, a cerebral vasospasm, hypoglycaemia, cardiac arrest, status epilepticus, perinatal asphyxia, anoxia, cerebral trauma, lathyrism, Alzheimer's disease, Parkinson's Disease and Huntington's disease, cerebral ischaemia or cerebral infarction, ischaemic, hypoxic or anoxic brain damage, spinal cord injury, tissue ischaemia and reperfusion injury in a mammal at risk for such damage.


SEQUENCE LISTING

<110> Sylentis S.A.U

<120> Methods and compositions to inhibit P2X7 receptor expression

<130> PC785537EPA

<140> 10171620.7
<141> 2005-08-30

<150> GB 0419295.1
<151> 2004-08-31

<150> GB 0504057.1
<151> 2005-02-28

<160> 109

<170> PatentIn version 3.3

<210> 1
<211> 19
<212> DNA
<213> Homo sapiens

<400> 1
caaagtcact cggatccag 19

<210> 2
<211> 19
<212> DNA
<213> Homo sapiens

<400> 2
agtcactcgg atccagagc 19

<210> 3
<211> 19
<212> DNA
<213> Homo sapiens

<400> 3
gtcactcgga tccagagca 19

<210> 4
<211> 19
<212> DNA
<213> Homo sapiens

<400> 4
ttatggcacc attaagtgg 19

<210> 5
<211> 19
<212> DNA
<213> Homo sapiens

<400> 5
gtggttcttc cacgtgatc 19

<210> 6
<211> 19
<212> DNA
<213> Homo sapiens

<400> 6
gctgtaccag cggaaagag                19

<210> 7
<211> 19
<212> DNA
<213> Homo sapiens

<400> 7
agagcctgtc atcagttct                19

<210> 8
<211> 19
<212> DNA
<213> Homo sapiens

<400> 8
gagcctgtca tcagttctg                19

<210> 9
<211> 19
<212> DNA
<213> Homo sapiens

<400> 9
ggtgaagggg atagcagag                19

<210> 10
<211> 19
<212> DNA
<213> Homo sapiens

<400> 10
ggggatagca gaggtgaaa                19

<210> 11
<211> 19
<212> DNA
<213> Homo sapiens

<400> 11
agaggagatc gtggagaat                19

<210> 12
<211> 19
<212> DNA
<213> Homo sapiens

<400> 12
gaggagatcg tggagaatg                19

<210> 13
<211> 19
<212> DNA
<213> Homo sapiens

<400> 13
tggagtgaag aagttggtg                19

<210> 14
<211> 19
<212> DNA
<213> Homo sapiens

<400> 14
gaagttggtg cacagtgtc                19

<210> 15

<211> 19
<212> DNA
<213> Homo sapiens

<400> 15
gttggtgcac agtgtcttt 19

<210> 16
<211> 19
<212> DNA
<213> Homo sapiens

<400> 16
ctctttcttc gtgatgaca 19

<210> 17
<211> 19
<212> DNA
<213> Homo sapiens

<400> 17
actttctcaa aacagaagg 19

<210> 18
<211> 19
<212> DNA
<213> Homo sapiens

<400> 18
ctttctcaaa acagaaggc 19

<210> 19
<211> 19
<212> DNA
<213> Homo sapiens

<400> 19
aacagaaggc caagagcag 19

<210> 20
<211> 19
<212> DNA
<213> Homo sapiens

<400> 20
acagaaggcc aagagcagc 19

<210> 21
<211> 19
<212> DNA
<213> Homo sapiens

<400> 21
cagaaggcca agagcagcg 19

<210> 22
<211> 19
<212> DNA
<213> Homo sapiens

<400> 22
ggccaagagc agcggttgt 19

<210> 23
<211> 19
<212> DNA
<213> Homo sapiens

<400> 23
aaagggatgg atggacccg                19

<210> 24
<211> 19
<212> DNA
<213> Homo sapiens

<400> 24
aagggatgga tggacccgc                19

<210> 25
<211> 19
<212> DNA
<213> Homo sapiens

<400> 25
agggatggat ggacccgca                19

<210> 26
<211> 19
<212> DNA
<213> Homo sapiens

<400> 26
aggaattcag accggaagg                19

<210> 27
<211> 19
<212> DNA
<213> Homo sapiens

<400> 27
ggaattcaga ccggaaggt                19

<210> 28
<211> 19
<212> DNA
<213> Homo sapiens

<400> 28
ttcagaccgg aaggtgtgt                19

<210> 29
<211> 19
<212> DNA
<213> Homo sapiens

<400> 29
ggtgtgtagt gtatgaagg                19

<210> 30
<211> 19
<212> DNA
<213> Homo sapiens

<400> 30
gggaaccaga agacctgtg                19

<210> 31
<211> 19
<212> DNA
<213> Homo sapiens

<400> 31
ccagaagacc tgtgaagtc                19

<210> 32

<211> 19
<212> DNA
<213> Homo sapiens

<400> 32
gacctgtgaa gtctctgcc                19

<210> 33
<211> 19
<212> DNA
<213> Homo sapiens

<400> 33
cagtgccgaa aacttcact                19

<210> 34
<211> 19
<212> DNA
<213> Homo sapiens

<400> 34
aacttcactg tgctcatca                19

<210> 35
<211> 19
<212> DNA
<213> Homo sapiens

<400> 35
acttcactgt gctcatcaa                19

<210> 36
<211> 19
<212> DNA
<213> Homo sapiens

<400> 36
cttcactgtg ctcatcaag                19

<210> 37
<211> 19
<212> DNA
<213> Homo sapiens

<400> 37
gaacaatatc gacttcccc                19

<210> 38
<211> 19
<212> DNA
<213> Homo sapiens

<400> 38
caatatcgac ttccccggc                19

<210> 39
<211> 19
<212> DNA
<213> Homo sapiens

<400> 39
tatcgacttc cccggccac                19

<210> 40
<211> 19
<212> DNA
<213> Homo sapiens

<400> 40
ctacaccacg agaaacatc                19

<210> 41
<211> 19
<212> DNA
<213> Homo sapiens

<400> 41
acatcctgcc aggtttaaa                19

<210> 42
<211> 19
<212> DNA
<213> Homo sapiens

<400> 42
catcctgcca ggtttaaac                19

<210> 43
<211> 19
<212> DNA
<213> Homo sapiens

<400> 43
acatcacttg taccttcca                19

<210> 44
<211> 19
<212> DNA
<213> Homo sapiens

<400> 44
catcacttgt accttccac                19

<210> 45
<211> 19
<212> DNA
<213> Homo sapiens

<400> 45
gactcagaat ccacagtgt                19

<210> 46
<211> 19
<212> DNA
<213> Homo sapiens

<400> 46
tccacagtgt cccattttc                19

<210> 47
<211> 19
<212> DNA
<213> Homo sapiens

<400> 47
acaggcgata atttttcag                19

<210> 48
<211> 19
<212> DNA
<213> Homo sapiens

<400> 48
caggcgataa tttttcaga                19

<210> 49

<211> 19
<212> DNA
<213> Homo sapiens

<400> 49
ttcagggcgg aataatggg                19

<210> 50
<211> 19
<212> DNA
<213> Homo sapiens

<400> 50
taatgggcat tgagatcta                19

<210> 51
<211> 19
<212> DNA
<213> Homo sapiens

<400> 51
tgggcattga gatctactg                19

<210> 52
<211> 19
<212> DNA
<213> Homo sapiens

<400> 52
cctagaccgt tggttccat                19

<210> 53
<211> 19
<212> DNA
<213> Homo sapiens

<400> 53
atacagtttc catcgcctt                19

<210> 54
<211> 19
<212> DNA
<213> Homo sapiens

<400> 54
atacagtttc cgtcgcctt                19

<210> 55
<211> 19
<212> DNA
<213> Homo sapiens

<400> 55
tacagtttcc gtcgccttg                19

<210> 56
<211> 19
<212> DNA
<213> Homo sapiens

<400> 56
gaccaccaac gtgtccttg                19

<210> 57
<211> 19
<212> DNA
<213> Homo sapiens

<400> 57
cgtgtccttg taccctggc                    19

<210> 58
<211> 19
<212> DNA
<213> Homo sapiens

<400> 58
cttcagatac gccaagtac                    19

<210> 59
<211> 19
<212> DNA
<213> Homo sapiens

<400> 59
aacaatgttg agaaacgga                    19

<210> 60
<211> 19
<212> DNA
<213> Homo sapiens

<400> 60
acaatgttga gaaacggac                    19

<210> 61
<211> 19
<212> DNA
<213> Homo sapiens

<400> 61
caatgttgag aaacggact                    19

<210> 62
<211> 19
<212> DNA
<213> Homo sapiens

<400> 62
tgttgagaaa cggactctg                    19

<210> 63
<211> 19
<212> DNA
<213> Homo sapiens

<400> 63
acggactctg ataaaagtc                    19

<210> 64
<211> 19
<212> DNA
<213> Homo sapiens

<400> 64
cggactctga taaaagtct                    19

<210> 65
<211> 19
<212> DNA
<213> Homo sapiens

<400> 65
aagtcttcgg gatccgttt                    19

<210> 66

<211> 19
<212> DNA
<213> Homo sapiens

<400> 66
agtcttcggg atccgtttt                19

<210> 67
<211> 19
<212> DNA
<213> Homo sapiens

<400> 67
gtcttcggga tccgttttg                19

<210> 68
<211> 19
<212> DNA
<213> Homo sapiens

<400> 68
atttgacatt atccagctg                19

<210> 69
<211> 19
<212> DNA
<213> Homo sapiens

<400> 69
tttgacatta tccagctgg                19

<210> 70
<211> 19
<212> DNA
<213> Homo sapiens

<400> 70
ccctctccta cttcggtct                19

<210> 71
<211> 19
<212> DNA
<213> Homo sapiens

<400> 71
ctgctgtcgc tcccatatt                19

<210> 72
<211> 19
<212> DNA
<213> Homo sapiens

<400> 72
gtgctgtcag ccctgtgtg                19

<210> 73
<211> 19
<212> DNA
<213> Homo sapiens

<400> 73
cgaatactac tacaggaag                19

<210> 74
<211> 19
<212> DNA
<213> Homo sapiens

<400> 74
tactactaca ggaagaagt                    19

<210> 75
<211> 19
<212> DNA
<213> Homo sapiens

<400> 75
gaagtgcgag tccattgtg                    19

<210> 76
<211> 19
<212> DNA
<213> Homo sapiens

<400> 76
gtgcgagtcc attgtggag                    19

<210> 77
<211> 19
<212> DNA
<213> Homo sapiens

<400> 77
agccgacatt aaagtatgt                    19

<210> 78
<211> 19
<212> DNA
<213> Homo sapiens

<400> 78
gccgacatta aagtatgtg                    19

<210> 79
<211> 19
<212> DNA
<213> Homo sapiens

<400> 79
agtatgtgtc ctttgtgga                    19

<210> 80
<211> 19
<212> DNA
<213> Homo sapiens

<400> 80
gtatgtgtcc tttgtggat                    19

<210> 81
<211> 19
<212> DNA
<213> Homo sapiens

<400> 81
tcccacatta ggatggtga                    19

<210> 82
<211> 19
<212> DNA
<213> Homo sapiens

<400> 82
ccagcagcta ctagggaga                    19

<210> 83

<211> 19
<212> DNA
<213> Homo sapiens

<400> 83
gtctgcaaga tgtcaaggg                19

<210> 84
<211> 19
<212> DNA
<213> Homo sapiens

<400> 84
gatgtcaagg gccaagaag                19

<210> 85
<211> 19
<212> DNA
<213> Homo sapiens

<400> 85
gggccaagaa gtcccaaga                19

<210> 86
<211> 19
<212> DNA
<213> Homo sapiens

<400> 86
gaagtcccaa gacctgcga                19

<210> 87
<211> 19
<212> DNA
<213> Homo sapiens

<400> 87
gtcccaagac ctgcgatgg                19

<210> 88
<211> 19
<212> DNA
<213> Homo sapiens

<400> 88
gacctgcgat ggacttcac                19

<210> 89
<211> 19
<212> DNA
<213> Homo sapiens

<400> 89
ccagaggaga tacagctgc                19

<210> 90
<211> 19
<212> DNA
<213> Homo sapiens

<400> 90
aggaggcgac tcctagatc                19

<210> 91
<211> 19
<212> DNA
<213> Homo sapiens

<400> 91
ggaggcgact cctagatcc                19

<210> 92
<211> 19
<212> DNA
<213> Homo sapiens

<400> 92
gctgcctccc atctcaact                19

<210> 93
<211> 19
<212> DNA
<213> Homo sapiens

<400> 93
ctccctgaga gccacaggt                19

<210> 94
<211> 19
<212> DNA
<213> Homo sapiens

<400> 94
gctggtcctg tccagacac                19

<210> 95
<211> 19
<212> DNA
<213> Homo sapiens

<400> 95
agagtttccg aagagtgaa                19

<210> 96
<211> 19
<212> DNA
<213> Homo sapiens

<400> 96
gagtttccga agagtgaag                19

<210> 97
<211> 19
<212> DNA
<213> Homo sapiens

<400> 97
gagtgaaggg cagtacagt                19

<210> 98
<211> 19
<212> DNA
<213> Homo sapiens

<400> 98
gggcagtaca gtggcttca                19

<210> 99
<211> 19
<212> DNA
<213> Homo sapiens

<400> 99
aggcctgctc tcttgaaca                19

<210> 100

<211> 19
<212> DNA
<213> Homo sapiens

<400> 100
ggcctgctct cttgaacag                    19

<210> 101
<211> 19
<212> DNA
<213> Homo sapiens

<400> 101
cttgaacttc acctgcaaa                    19

<210> 102
<211> 19
<212> DNA
<213> Homo sapiens

<400> 102
attgagacag tgcacatga                    19

<210> 103
<211> 19
<212> DNA
<213> Homo sapiens

<400> 103
ggtgtgtagt gcatgaagg                    19

<210> 104
<211> 19
<212> DNA
<213> Homo sapiens

<400> 104
gagattctct tttccatgc                    19

<210> 105
<211> 19
<212> DNA
<213> Homo sapiens

<400> 105
aatggtttgg agaaggaag                    19

<210> 106
<211> 19
<212> DNA
<213> Homo sapiens

<400> 106
atggtttgga gaaggaagt                    19

<210> 107
<211> 19
<212> DNA
<213> Homo sapiens

<400> 107
tggtttggag aaggaagtg                    19

<210> 108
<211> 19
<212> DNA
<213> Homo sapiens

<400> 108
ctacaccaca tacgccaag                19

<210> 109
<211> 19
<212> DNA
<213> Homo sapiens

<400> 109
ttcagatacg ccaagtact                19